# EUROPÄISCHE PATENTSCHRIFT

(11) **EP 1 292 607 B1**
(45) Veröffentlichungstag und Bekanntmachung des Hinweises auf die Patenterteilung: **04.06.2008**
(21) Anmeldenummer: 01929632.6
(22) Anmeldetag: 09.05.2001
(51) Int. Cl.: C07J 41/00, A61K 31/565, A61P 5/36

(54) **NEUE FESTKÖRPERFORMEN DES MESOPROGESTINS 11BETA- 4E-(HYDROXYIMINOMETHYL)-PHENYL -17ALPHA-METHOXYMETHYL-17BETA-METHOXY-ESTRA-4,9-DIEN-3-ON**
NOVEL SOLID BODY FORMS OF MESOPROGESTIN 11BETA- 4E-(HYDROXYIMINOMETHYL)-PHENYL -17ALPHA-METHOXYMETHYL-17BETA-METHOXY-ESTRA-4,9-DIEN-3-ONE
NOUVELLES FORMES SOLIDES DE MESOPROGESTINE 11BETA- 4E-(HYDROXYIMINOMETHYL)-PHENYLE -17ALPHA-METHOXYMETHYLE-17BETA-METHOXY-ESTRA-4,9-DIENE-3-ONE

(30) Priorität: 23.05.2000 EP 00110887
(43) Veröffentlichungstag der Anmeldung: 19.03.2003
(73) Patentinhaber: Bayer Schering Pharma Aktiengesellschaft, 13353 Berlin (DE)
(72) Erfinder: GRAWE, Detlef, 99510 Kleinromstedt (DE); HÖSEL, Peter, 07745 Jena (DE); MÜLLER, Uwe, 07745 Jena (DE); WINTER, Gabriele, 16567 Schönfliess (DE)
(74) Vertreter: Ziebig, Marlene
(86) Internationale Anmeldenummer: PCT/EP2001/005237
(87) Internationale Veröffentlichungsnummer: WO 2001/090137

(56) Entgegenhaltungen:
- EP-A- 0 648 778
- WO-A-97/32890

## Beschreibung

Die vorliegende Erfindung betrifft eine neue Festkörperform des Mesoprogestins 11β-[4E-(Hydroxylminomethyl)phenyl]-17α-methoxymethyl-17β-methoxy-estra-4,9-dien-3-on (Oxim J 867), insbesondere eine hochreine und stabile amorphe Form der Verbindung J 867, Verfahren zu dessen Herstellung sowie dessen Verwendung in pharmazeutischen Zusammensetzungen.

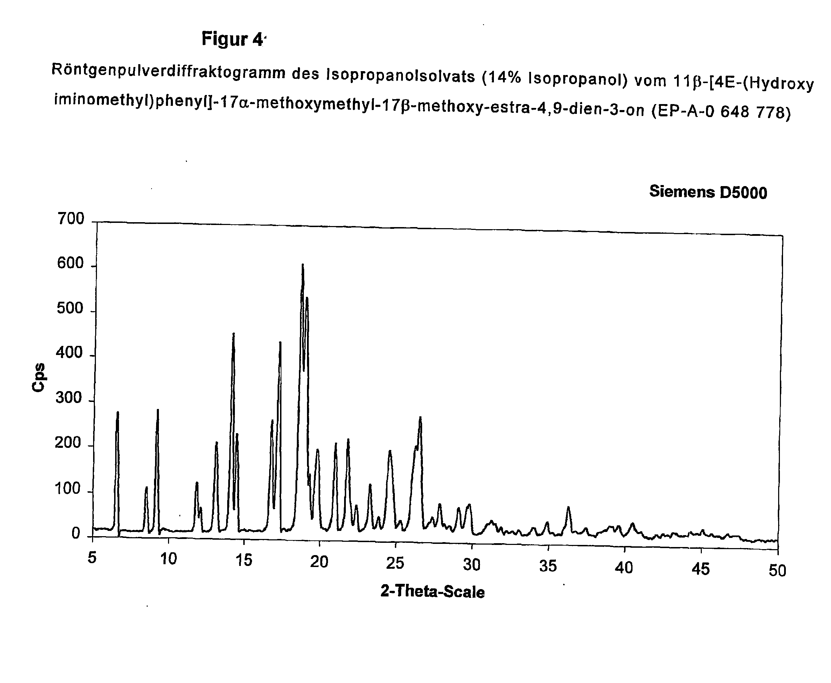

11β-[4-(Hydroxyiminomethyl)phenyl]-17α-methoxymethyl-17β-methoxy-estra-4,9-dien-3-on ist beispielsweise aus EP-A-0 648 778 bzw. DE-A-43 32 283 bekannt. Die dort nach Umkristallisation aus Methylenchlorid/Isopropanol erhaltene Form schmilzt bei 118 °C. Bei der beschriebenen Kristallform handelt es sich um ein Isopropanol-Solvat mit 14% Isopropanol (siehe XRPD: Tabelle 2/Figur 4). Kennzeichnend für das Oxim ist seine starke Tendenz aus Lösungsmitteln als Solvat auszukristallisieren. Die Solvatform aber ist für pharmazeutische Anwendungen wenig geeignet. Die Lösungsmittelbindung an die Substanz ist sehr fest und wird erst bei höheren Temperaturen gelöst. Die Oxim-Solvate spalten im allgemeinen erst oberhalb 100°C das Lösemittel vollständig ab. Dabei entstehen je nach Heizrate inhomogene amorph-kristalline Mischformen.

Die Reinigung des Oxims von Nebenprodukten der Oximierungsreaktion (Dioxime, Z-Oxim) erfolgt durch aufwendige Säulenchromatographie mit Toluol/Acetongradienten. Eine Reinigung durch Kristallisationsverfahren wird durch die allgemein schlechten Löslichkeiten in den dafür in Frage kommenden Lösungsmitteln erschwert. Einschränkend kommt hinzu, daß bei höheren Temperaturen in Lösung eine Isomerisierung des E-Isomeren der Verbindung zum Z-Isomeren auftritt.

Aufgabe der Erfindung ist daher durch ein geeignetes Verfahren die aufwendige Säulenchromatographie zu ersetzen und eine homogene, lösungsmittelfreie und stabile feste Form mit guten pharmazeutischen Eigenschaften bereitzustellen.

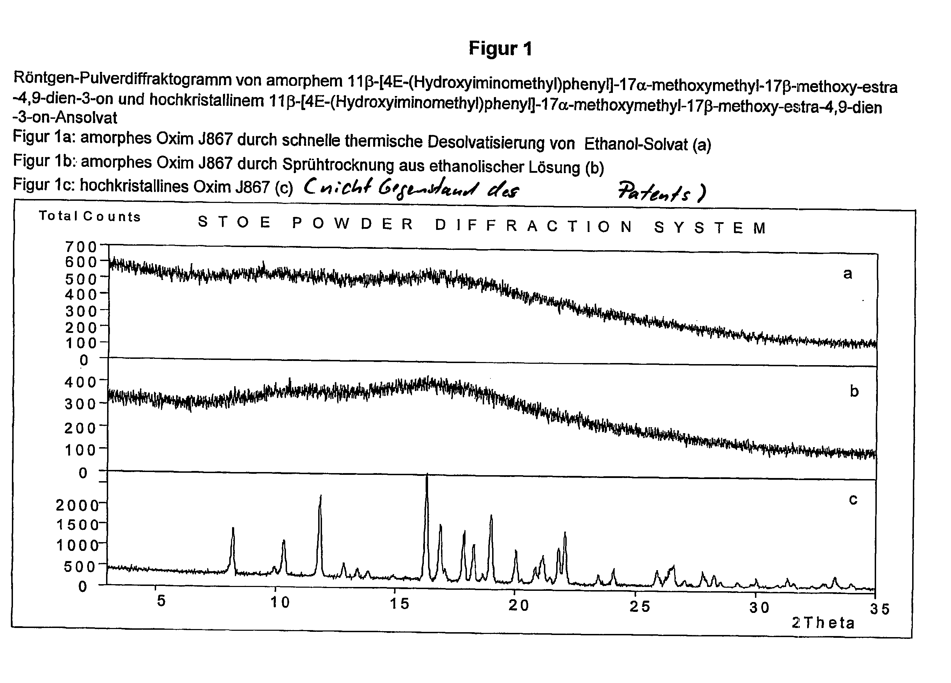

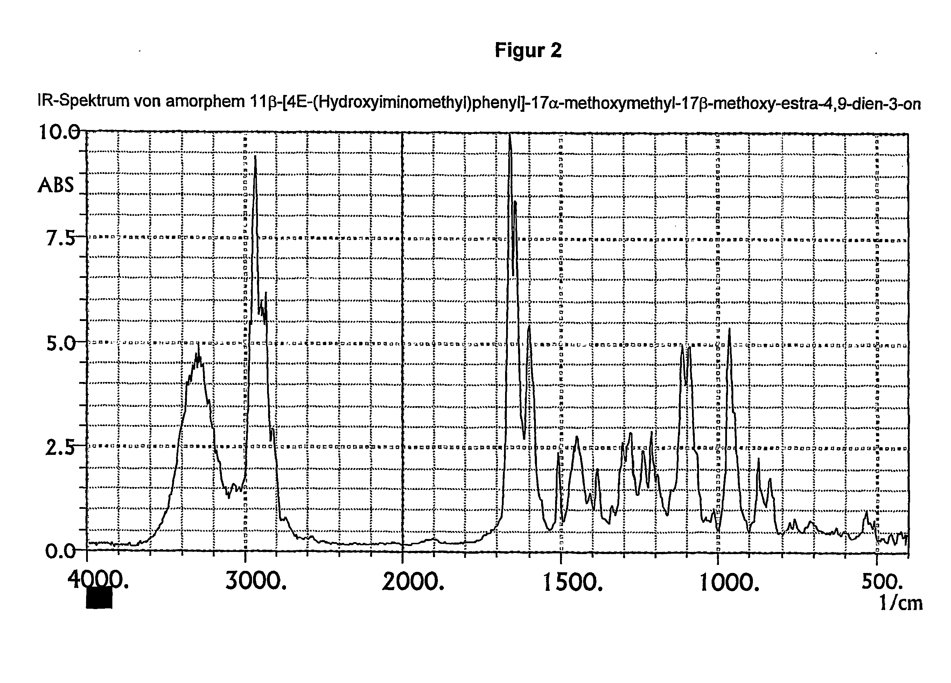

Diese Aufgabe wird gelöst durch amorphes 11β-[4E-(Hydroxyiminomethyl)phenyl]-17α-methoxymethyl-17β-methoxy-estra-4,9-dien-3-on, das dadurch gekennzeichnet ist, daß keine kristallinen Reflexe im Röntgen-Pulverdiffraktogramm (XRPD; Figur 1a/b) zu sehen sind, bzw. dessen Modifikation durch das in Figur 2 dargestellte IR-Spektrum gekennzeichnet ist.

Kristalline Solvate von 11β-[4E-(Hydroxyiminomethyl)phenyl]-17α-methoxymethyl-17β-methoxy-estra-4,9-dien-3-on können mittels einem Verfahren hergestellt werden, umfassend die folgenden Schritte:
a) Herstellung eines Solvats von 11β-[4E-(Hydroxyiminomethyl)phenyl]17α-methoxymethyl-17β-methoxy-estra-4,9-dien-3-on und Auflösen des Solvats oberhalb der Sättigungslöslichkeit in einem Lösungsmittel, insbesondere Toluol, das unterschiedlich vom Solvat-Lösungsmittel ist und in welchem die Solvat-Kristallstruktur instabil ist,
b) gegebenenfalls Zugabe eines weiteren Lösungsmittels als Rekristallisations-Inhibitor, insbesondere Methanol oder Essigester, und
c) Zugabe eines Solvatbildner-Lösungsmittels, insbesondere Ethanol, Aceton und Methyl-tert.-butylether (MtBE) und Kristallisieren des Solvats.

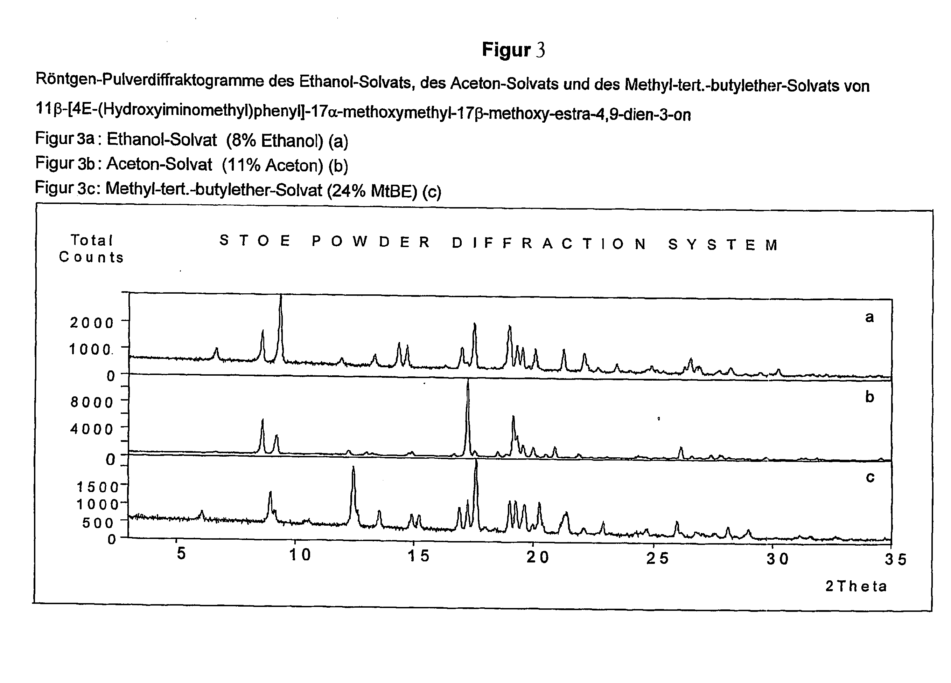

Die kristallinen Solvate von 11β-[4E-(Hydroxyiminomethyl)phenyl]-17α-methoxymethyl-17β-methoxy-estra-4,9-dien-3-on, nämlich das Methyl-tert.-butylether-Solvat, das Aceton-Solvat und das Ethanol-Solvat, wobei die hochkristallinen Formen durch die in Figur 3a/b/c und Tabelle 1 dargestellten Röntgen-Pulverdiffraktogramme gekennzeichnet sind, sind neu und stellen Zwischenstufen auf dem Weg zum amorphen 11β-[4E-(Hydroxyiminomethyl)phenyl]-17α-methoxymethyl-17β-methoxy-estra-4,9-dien-3-on.

Durch Trocknen der vorstehend erhaltenen, kristallinen Solvate von 11β-[4E-(Hydroxyiminomethyl)phenyl]-17α-methoxymethyl-17β-methoxy-estra-4,9-dien-3-on, insbesondere durch thermische Desolvatisierung vorzugsweise unter Vakuum und mit einer Heizrate im Produkt von mindestens 0,5°C/min oder aus einer Lösung des Solvats durch Sprühtrocknung (XRPD; Figur 1) unterhalb des Glasübergangspunktes der amorphen Struktur wird dann die amorphe Form von 11β-[4E-(Hydroxyiminomethyl)phenyl]-17α-methoxymethyl-17β-methoxy-estra-4,9-dien-3-n gewonnen. Gegenstand der vorliegenden Erfindung sind auch pharmazeutische Zusammensetzungen, insbesondere feste pharmazeutische Zusammensetzungen, umfassend das vorstehend beschriebene amorphe 11β-[4E-(Hydroxyiminomethyl)phenyl]-17α-methoxymethyl-17β-methoxy-estra-4,9-dien-3-on, in Kombination mit einem pharmazeutisch verträglichen Träger und/oder Verdünnungsmittel.

Die pharmazeutischen Zusammensetzungen der vorliegenden Erfindung werden mit den üblichen festen oder flüssigen Trägerstoffen oder Verdünnungsmitteln und den üblicherweise verwendeten pharmazeutisch technischen Hilfsstoffen entsprechend der gewünschten Applikationsart mit einer geeigneten Dosierung in an sich bekannter Weise hergestellt.
In den Figuren 1a und b sind die Röntgen-Pulverdiffraktogramme von amorphem 11β-[4E-(Hydroxyiminomethyl)phenyl]-17α-methoxymethyl-17β-meth-oxy-estra-4,9-dien-3-on dargestellt.
In Figur 2 ist ein IR-Spektum von amorphem 11β-[4E-(Hydroxy-iminomethyl)phenyl]-17α-methoxymethyl-17β-methoxy-estra-4,9-dien-3-on dargestellt.
In Figur 3 (Tabelle 1) sind Röntgen-Pulverdiffraktogramme des Methyl-tert.-butylether-Solvats, des Aceton-Solvats, des Ethanol-Solvats und des Isopropanol-Solvats (vgl. Tabelle 1) von 11β-[4E-(Hydroxyiminomethyl)phenyl]-17α-methoxymethyl-17β-methoxy-estra-4,9-dien-3-on dargestellt,
In Figur 5 ist ein Röntgen-Pulverdiffraktogramm von amorphem 11β-[4E-(Hydroxyiminomethyl)phenyl]-17α-methoxymethyl-17β-methoxy-estra-4,9-dien-3-on zum Zeitpunkt 0 (Start), nach 12 Monaten bei 25 °C und nach 12 Monaten bei 40 °C dargestellt.
In Figur 6 ist die Löslichkeit von hochkristallinem 11β-[4E-(Hydroxyiminomethyl)phenyi]-17α-methoxymethy)-17β-methoxy-estra-4,9-dien-3-on-Ansolvat (nicht Gegenstand des Patents) bzw. amorphem 11β-[4E-(Hydroxyiminomethyl)phenyl]-17α-methoxymethyl-17β-methoxy-estra-4,9-dien-3-on in Wasser mit und ohne Tensid gezeigt.

**Tabelle 1: XRPD-Daten der Solvate des Oxims J867 - d-Werte (d_{obs}) and relative Intensitäten (l_{obs}) der stärksten Peaks**

| **Ethanol-Solvat** | | **Methyl tert.-butyl ether- Solvat** | | **Aceton- Solvat** | | **Isopropanol-Solvat** | |
|---|---|---|---|---|---|---|---|
| **d_{obs} [Å]** | **l_{obs}** | **d_{obs} [Å]** | **l_{obs}** | **d_{obs} [Å]** | **l_{obs}** | **d_{obs} [Å]** | **l_{obs}** |
| 13,22 | 19,3 | 14,47 | 13,7 | 10,24 | 42,1 | 13,42 | 52,1 |
| 10,27 | 44,4 | 9,86 | 43,9 | 9,58 | 22,8 | 10,34 | 17,8 |
| 9,44 | 100,0 | 9,67 | 18,3 | 7,21 | 5,9 | 9,61 | 51,3 |
| 7,39 | 8,9 | 8,36 | 6,6 | 6,81 | 3,3 | 7,48 | 21,4 |
| 6,64 | 16,6 | 7,10 | 81,7 | 6,68 | 2,0 | 7,33 | 15,6 |
| 6,17 | 39,6 | 6,53 | 22,6 | 5,99 | 3,2 | 6,76 | 36,4 |
| 6,03 | 34,4 | 5,94 | 20,5 | 5,94 | 5,0 | 6,28 | 80,2 |
| 5,21 | 35,6 | 5,82 | 19,5 | 5,32 | 3,2 | 6,13 | 45 |
| 5,06 | 68,7 | 5,24 | 32,3 | 5,14 | 100,0 | 5,29 | 51,6 |
| 4,67 | 72,3 | 5,14 | 42,5 | 5,05 | 6,8 | 5,14 | 69,1 |
| 4,59 | 40,8 | 5,04 | 100,0 | 4,79 | 5,8 | 4,77 | 100,0 |
| 4,54 | 30,3 | 4,92 | 5,6 | 4,70 | 3,3 | 4,69 | 94,4 |
| 4,48 | 6,3 | 4,67 | 42,9 | 4,63 | 51,7 | 4,60 | 22,1 |
| 4,42 | 30,0 | 4,60 | 42,6 | 4,59 | 26,0 | 4,49 | 31,5 |
| 4,17 | 33,5 | 4,52 | 38,5 | 4,53 | 14,3 | 4,23 | 39,7 |
| 4,02 | 25,8 | 4,44 | 9,7 | 4,44 | 11,3 | 4,08 | 38,3 |
| 3,91 | 6,9 | 4,38 | 1,7 | 4,32 | 3,6 | 3,97 | 14,9 |
| 3,78 | 12,5 | 4,16 | 28,8 | 4,25 | 12,6 | 3,82 | 23,8 |
| 3,70 | 4,5 | 4,02 | 9,2 | 4,06 | 4,7 | 3,73 | 11,6 |
| 3,59 | 7,8 | 3,88 | 15,9 | 3,65 | 2,7 | 3,62 | 37,1 |
| 3,57 | 13,3 | 3,66 | 5,0 | 3,63 | 2,1 | 3,59 | 22,4 |
| 3,54 | 7,0 | 3,63 | 5,4 | 3,40 | 14,6 | 3,41 | 35,0 |
| 3,50 | 4,2 | 3,59 | 9,6 | 3,34 | 3,5 | 3,39 | 35,9 |
| 3,38 | 10,0 | 3,42 | 20,5 | 3,30 | 2,1 | 3,36 | 37,61 |
| 3,35 | 23,9 | 3,32 | 7,8 | 3,25 | 4,8 | 3,26 | 11,8 |
| 3,32 | 12,5 | 3,29 | 5,5 | 3,20 | 5,0 | 3,2 | 14,0 |
| 3,30 | 12,7 | 3,23 | 6,4 | 3,16 | 2,0 | 3,07 | 16,4 |
| 3,21 | 6,3 | 3,17 | 14,5 | 3,00 | 3,4 | 3 | 13,6 |
| 3,15 | 10,7 | 3,07 | 12,2 | 2,86 | 2,1 | 2,47 | 12,65 |
| 3,02 | 4,9 | 2,87 | 5,6 | 2,85 | 2,2 | 2,23 | 10,6 |
| 2,98 | 3,7 | 2,83 | 4,6 | 2,81 | 2,2 | | |
| 2,95 | 10,6 | | | 2,59 | 2,4 | | |

Die Erfindung betrifft eine neue hochreine und stabile amorphe Form des Mesoprogestins 11β-[4E-(Hydroxyiminomethyl)phenyl]-17α-methoxymethyl-17β-methoxy-estra-4,9-dien-3-on (J 867): 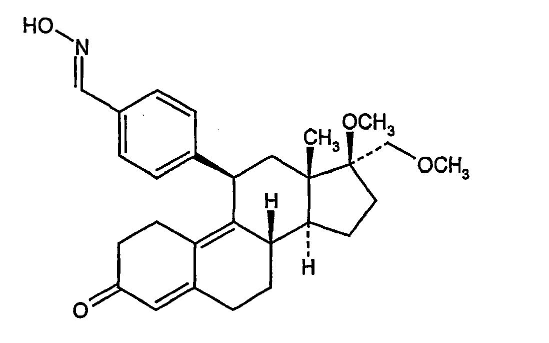 und Solvat-Formen von J867, die Zwischenprodukte zur Herstellung der amorphen Form darstellen. Als Problem bei der Herstellung der amorphen bzw. hochkristallinen Form hat sich erwiesen, daß die Substanz im Stadium der Desolvatisierung ein amorphes Zwischenstadium durchläuft, aus dem je nach Heizrate, Endtemperatur und Trocknungsdauer mehr oder weniger vollständig die Ansolvat/Anhydrat-Form rekristallisiert. Der Grad der Kristallinität ist jedoch schwer zu steuern, so daß inhomogene Mischformen aus amorphen und kristallinen Anteilen entstehen. Außerdem führt die thermische Belastung der zur Z-Isomerisierung und Oxim-Spaltung neigenden chemischen Struktur zu einer Verschlechterung der Reinheit.

Die Herstellung der für die feste Endform des Oxims geeigneten Solvatform kann erfindungsgemäß mit der Abtrennung des Z-Isomeren und der Dioxime verbunden werden. Durch eine gezielte Ausnutzung der thermodynamischen Instabilität geeigneter Solvate, vorzugsweise des Solvats des Methyl-tert.-butylethers (MTBE), in einer dafür geeigneten Lösemit telumgebung, ist eine vollständige Auflösung des Solvatgitters weit oberhalb der Löslichkeitsgrenze und schon in der Nähe der Raumtemperatur möglich. Im Kontakt mit Toluen im Massenverhältnis von 1:2 bis 1:1 löst sich die Kristallform des MTBE-Solvats, trotz der nur geringen Löslichkeit in Toluol von nur 3,3 Gew%, schon bei 20-35°C schnell und vollständig auf. Es erfolgt ein sogenanntes kaltes Schmelzen. Dieser Zustand kann durch Zugabe geeigneter Lösungsmittel als Rekristallisations-Inhibitoren für technische Anwendungen stabilgehalten werden. So kann der Übersättigungszustand in toluenischer Lösung durch Zugabe von nur 5 Vol.-% Methanol für Stunden stabilisiert werden. Anschließend wird durch Zugabe des Solvatbildner-Lösungsmittels (MTBE) in geeigneter Konzentration (z.B. 100 - 130 Vol.-% MTBE, bezogen auf eingesetztes Toluen) das Solvatgitter wieder aufgebaut, wobei die Verunreinigungen größtenteils in Lösung bleiben.

Durch ein besonders schnelles Trocknungsverfahren, vorzugsweise Sprühtrocknung, unterhalb des Glasübergangspunktes direkt aus der Lösung des Oxim-Solvates J867 in einem organischen Lösungsmittel, vorzugsweise Ethanol, kann eine stabile, vollständig amorphe, lösungsmittelfreie Struktur gewonnen werden. Hierzu wird in Ethanol zu 3 bis 13 Gew.-% gelöstes Oxim über ein geeignetes Zerstäubungsaggregat (Zweistoffdüse oder Zentrifugalzerstäuber) in heißem Stickstoff versprüht und in extrem kurzer Zeit zu amorphen, mikrofeinen Partikeln getrocknet. Die Eintrittstemperatur des Stickstoffs beträgt dabei 120°C bis 200°C, vorzugsweise 150°C-180°C. Das Verhältnis Oximlösung zu Trocknungsgas sollte dabei 0,01 bis 0,3 kg Lösung /m³ N₂, vorzugsweise 0,08 bis 0,12 kg Lösung /m³ N₂, betragen. Der Trocknungsprozeß wird so gesteuert, daß im Trocknerausgang eine Temperatur von 55°C bis 95°C, vorzugsweise 75 bis 85°C, eingestellt wird. Diese Temperatur liegt deutlich unter der Glasübergangstemperatur des amorphem Oxims (105°C), wo die Substanz eine gel/glasartige Konsistenz annimmt und oberhalb derer eine mehr oder weniger schnelle bzw. spontane Rekristallisation stattfinden kann.

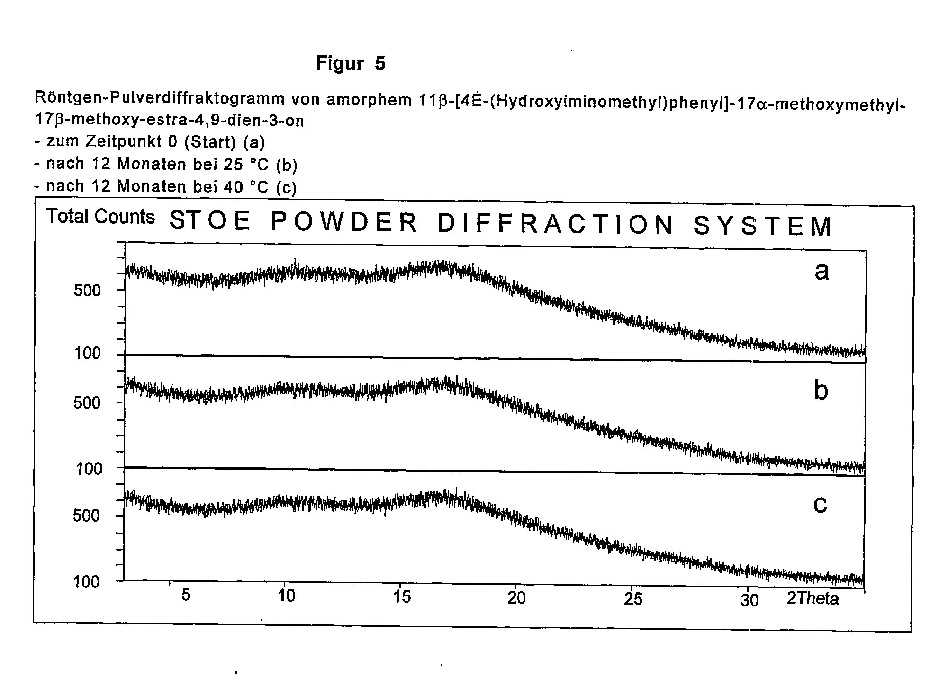

Durch Anwendung der Sprühtrocknung wird eine lösungsmittelfreie, homogene und mikrofeine Festkörperform von Oxim J 867 in einem Verfahrensschritt erhalten.
Der Vorteil der Verwendung der erfindungsgemäßen amorphen Form von Oxim J867 zur Herstellung von Arzneimitteln liegt darin, daß diese amorphe Struktur neben den guten Löslichkeitseigenschaften überraschenderweise eine sehr gute Lagerstabilität aufweist. Auch nach 12 Monaten im accelerated ICH-Stability-Test (40°C, 75% relative Feuchtigkeit) zeigt die amorphe Struktur keine Anzeichen einer Rekristallisation und/oder chemischen Zersetzung (siehe Figur 5). Auch während der pharmazeutischen Verarbeitung (Feuchtgranulierung, Tablettierung) bleibt die physikalische Struktur des Oxims stabil. Das heißt, eine Umwandlung der amorphen in die hochkristalline Form bzw. eine E/Z-Isomerisierung tritt nicht auf. Durch die Stabilität werden negative Einflüsse derartiger Umwandlungen auf die Bioverfügbarkeit des Wirkstoffes vermieden.

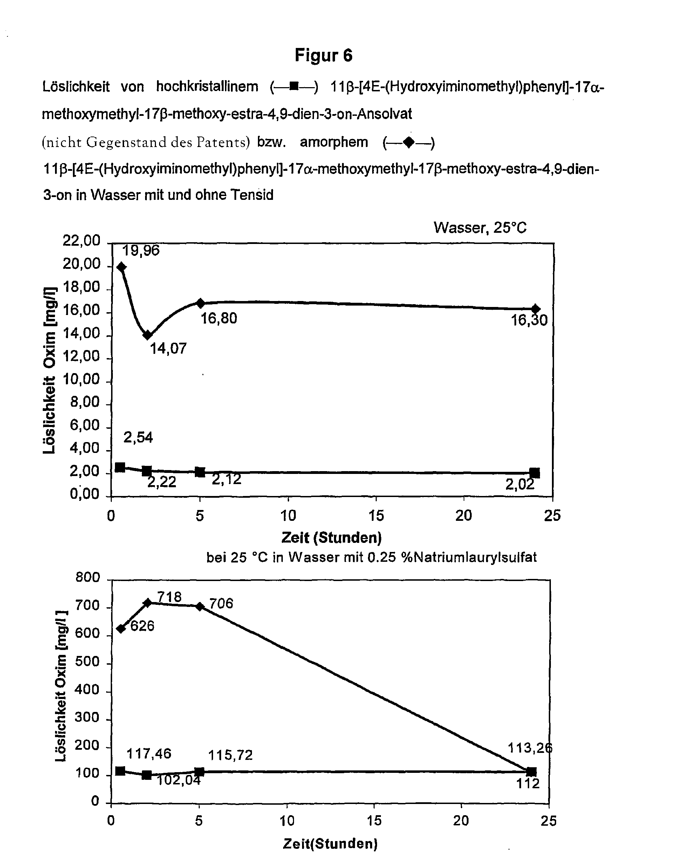

Des weiteren besitzt die amorphe Form hervorragende Löslichkeitseigenschaften. So ist die Sättigungslöslichkeit in Wasser als auch in Tensidlösung (0,25 %-iges Natriumlaurylsulfat) etwa 7 bis 8 mal höher als die der hochkristallinen Form, wobei dieses hohe Übersättigungsninevau für mehrere Stunden, wie im Falle von Wasser für über 24 h stabil bleibt (siehe Figur 6).

Mit dem erfindungsgemäßen Verfahren, insbesondere der thermischen Desolvatisierung ist es grundsätzlich möglich, je nach Prozeßbedingungen amorph-kristalline Mischformen zu erzeugen, die jedoch nicht reproduzierbar erhältlich und für die Entwicklung eines validierten Herstellungsverfahrens für die Qualitätskontrolle, für die pharmazeutische Weiterverarbeitung und für die Zulassung erhebliche Probleme darstellen können.

Das Oxim J867 ist gemäß EP-A-0 648 778 bzw. DE-A-43 32 283 eine antigestagen wirkende Substanz, die bei gleicher Aktivität wie RU 486 (Mifepriston) am Progesteron-Rezeptor eine im Vergleich mit RU 8486 deutlich reduzierte antiglucocortikoide Aktivität besitzt.
In den US-Anmeldungen Nr. 09/386,141, 09/386,140 und 09/386,133 wird das Oxim J 867 als Mesoprogestin bezeichnet. Mesoprogestine sind hierbei als Verbindungen definiert, die in vivo sowohl agonistische als auch antagonistische Aktivität am Progesteron-Receptor (PR) aufweisen. Entsprechende funktionale Zustände können mit Gestagen und Antigestagen nicht erreicht werden.
Das Oxim J 867 eignet sich insbesondere für folgende Verwendungen:
a) Das Oxim J 867 kann gemäß US-Anmeldung Nr. 09/386,133, gegebenenfalls zusammen mit einem Estrogen, zur Herstellung eines Arzneimittels zur weiblichen Kontrazeption verwendet werden.
b) Das Oxim J867 kann gemäß US-Anmeldung Nr. 09/386,141 zur Behandlung und Verhinderung von benignen hormonabhängigen gynäkologischen Störungen; wie
   - zur Behandlung von gynäkologischen Störungen, wie Endometriose, uterinen Fibroiden, postoperativen peritonealen Adhäsionen, dysfunktionaler Blutung (Metrorrhagie, Menorrhagie) and Dysmenorrhöe;
   - zur Verhinderung von gynäkologischen Störungen; wie postoperativen, peritonealen Adhäsionen, dysfunktionaler Uterusblutung (Metrorrhagie, Menorrhagie) und Dysmenorrhöe, verwendet werden. Die tägliche Dosis des Mesoprogestins beträgt 0,5 bis 100 mg, vorzugsweise 5,0 bis 50 mg und am stärksten bevorzugt 10 bis 25 mg.
c) Das Oxim J867 kann gemäß US-Anmeldung Nr. 09/386,140 ebenso als pharmazeutischer Bestandteil zur Herstellung eines Arzneimittels gegebenenfalls zusammen mit einem Estrogen in der Hormon-Replacement-Therapy (HRT) und zur Behandlung von Hormonmangel und Symptomen von Hormon-Irregularität eingesetzt werden.

### Meßverfahren

### Röntgenpulverdiffraktometrie (X-Ray Powder Diffraction; XRPD):

Die Daten wurden mit einem STOE Powder Diffraktometer STADIP mit Germanium-monochromatischer CuKα₁-Strahlung (λ = 1.540598 Å) - zwischen 3° ≤ 2Θ ≤ 35° bzw. einem Siemens Diffraktometer D5000 mit Cu-Anode (5° ≤ 2Θ ≤ 50°) ermittelt.

### IR-Spektroskopie:

Verwendet wurde ein NICOLET 20 SXB mit Photoacoustic Detector MTEC (KBr, 8t, 90 Sekunden).

### Löslichkeitsuntersuchungen:

200 mg Substanz wurden bei 25°C in 50 ml Wasser (ohne und mit 0,25% SDS (Natriumlaurylsulfat)) gegeben. Proben wurden nach 0,5 h, 2 h, 5 h und 24 h genommen.
Probenahme: 0,45 µm Filtervorsatz, Verdünnung 1:1 mit MeOH, HPLC.

### HPLC:

Die Reinheitsbestimmung erfolgt nach folgender Methode:
Säule: Superspher Si 60, 250x4 mm, 4 µm
Eluent: Chloroform (3% Ethanol)/Wasser wie 96,9/0,1
Fluß: 1 ml/min
Detektion: UV (299 nm)
Auswertung: 100%-Flächennormalisierung

### Headspace für Restlösungsmittel:

GC-Autosystem mit HS40 Perkin Elmer, Säule DB-Wax, 30 m x 0,23 mm, FID.

### Korngrößenverteilung:

Sympatec HELOS (H0445), Trockendispergiersystem(RODOS), Druck 2 bar.

### DSC:

Perkin Eimer DSC 7 und NETZSCH DSC 200/1/F in Pan-Al, pierced lid, Stickstoff.

Die Erfindung wird durch die nun folgenden Beispiele näher erläutert.

### Beispiele

### Beispiel 1

2 kg Oxim J867 (MtBE-Solvat mit 20 bis 24 % MtBE; soweit nicht anders angegeben, sind die in den Solvaten enthaltenen Prozent an Lösungsmittel Gewichtsprozent) werden in 20 1 Ethanol bei 50°C unter Rühren gelöst. Die Lösung wird in einem unter inerten Bedingungen (Stickstoff) betriebenen Sprühtrockner im Gleichstromverfahren sprühgetrocknet. Die Trockenkammer des Sprühtrockners wird mit 60 m³/h Stickstoff, der auf 175°C vorgeheizt ist, beschickt. Anschließend wird über eine Schlauchpumpe die Lösung mit 6,4 l/h über eine Zweistoffdüse, die mit 3 bar Stickstoff als Treibgas betrieben wird, in der Trockenkammer versprüht. Die Trocknungsgasaustrittstemperatur beträgt 79°C - 83°C. Das zu mikrofeinen Partikeln getrocknete Oxim J867 wird in einem Produktfilter vollständig aufgefangen.

Das Oxim J867 weist folgende Qualitätsparameter auf:

| | |
|---|---|
| Restlösemittel | 0,44% Ethanol, 0,11% MtBE |
| Wasser | 0,5% |
| Korngröße | 100% < 12,5 µm |
| | 50% < 2,25 µm |
| | 10% < 0.82 µm |
| Reinheit (Gehalt an Z-Oxim) | keine Z-Isomerisierung während der thermischen Belastung: 1,7 F% (Flächenprozent) vor Trocknung 1,7 F% nach Trocknung |
| XRPD | 100% amorph - keine kristallinen Reflexe |

### Beispiel 2

Zu 20 g Oxim J867 (MTBE-Solvat mit 20% - 24% MtBE) werden 50 ml Toluen gegeben. Die Substanz löst sich bei 25°C sehr schnell und vollständig auf. Zur Lösung gibt man 2,7 ml Methanol und anschließend versetzt man die Lösung mit 65 ml MtBE. Nach etwa 1 min beginnt die Rekristallisation des MtBE-Solvates. Der gesamte Prozeß verläuft bei Raumtemperatur. Die Suspension wird 1 h bei 5°C gerührt, dann filtriert und der Filterkuchen mit 30 ml kaltem MtBE gewaschen. Nach der Trocknung beträgt die Ausbeute 84 Gew.-% des Einsatzmaterials. Das erhaltene Produkt ist wiederum ein MTBE-Solvat mit 22,3 % MtBE.Der Reinigungseffekt bezüglich der Hauptverunreinigungen wird in folgender Tabelle sichtbar:

| | Einsatzmaterial | Rekristallisat |
|---|---|---|
| E-Oxim | 97,7 F% | 98,8 F% |
| Z-Oxim | 1,7 F% | 1,0 F% |
| Dioxim | 0,4 F% | 0,04 F% |

## Patentansprüche

1. Amorphes 11β-[4E-(Hydroxyiminomethyl)phenyl]-17α-methoxymethyl-17β-methoxy-estra-4,9-dien-3-on, **dadurch gekennzeichnet, dass** keine kristallinen Reflexe im Röntgen-Pulverdiffraktogramm (XRPD) zu sehen sind.

2. Kristalline Solvate von 11β-[4E-(Hydroxyiminomethyl)phenyl]-17α-methoxymethyl-17β-methoxy-estra-4,9-dien-3-on, nämlich das Methyl-tert.-butylether-Solvat, wobei die kristalline Form durch das in Figur 3c dargestellte Röntgen-Pulverdiffraktogramm **gekennzeichnet** ist, das Aceton-Solvat, wobei die kristalline Form durch das in Figur 3b dargestellte Röntgen-Pulverdiffraktogramm **gekennzeichnet** ist, und das Ethanol-Solvat, wobei die kristalline Form durch das in Figur 3a dargestellte Röntgen-Pulverdiffraktogramm **gekennzeichnet** ist..

3. Verfahren zur Herstellung eines kristallinen Solvats von 11β-[4E-(Hydroxyiminomethyl)phenyl]-17α-methoxymethyl-17β-methoxy-estra-4,9-dien-3-on nach Anspruch 2 umfassend die folgenden Schritte:
a) Herstellung eines Solvats von 11β-[4E-(Hydroxyiminomethyl)phenyl]-17α-methoxymethyl-17β-methoxy-estra-4,9-dien-3-on und Auflösen des Solvats oberhalb der Sättigungslöslichkeit in einem Lösungsmittel, das unterschiedlich vom Solvat-Lösungsmittel ist und in welchem die Solvat-Kristallstruktur instabil ist,
b) gegebenenfalls Zugabe eines weiteren Lösungsmittels als Rekristallisations-Inhibitor und
c) Zugabe eines Solvatbildner-Lösungsmittels und Kristallisieren des Solvats von 11β-[4E-(Hydroxyiminomethyl)phenyl]-17α-methoxymethyl-17β-methoxy-estra-4,9-dien-3-on.

4. Verfahren zur Herstellung von amorphem 11β-[4E-(Hydroxyiminomethyl)phenyl]-17α-methoxymethyl-17β-methoxy-estra-4,9-dien-3-on durch Trocknung des nach Anspruch 3 erhaltenen kristallinen Solvats unterhalb des Glasübergangspunkts der amorphen Substanz, wobei die Trocknung des Solvats während der Desolvatisierungsphase mit einer Heizrate im Produkt von mindestens 0,5°C/min oder aus einer Lösung des Solvats durch Sprühtrocknung erfolgt.

5. Pharmazeutische Zusammensetzung, umfassend amorphes 11β-[4E-(Hydroxyiminomethyl)phenyl]-17α-methoxymethyl-17β-methoxy-estra-4,9-dien-3-on nach Anspruch 1 in Kombination mit einem pharmazeutisch verträglichen Träger und/oder Verdünnungsmittel.

6. Amorphes 11β-[4E-(Hydroxyiminomethyl)phenyl]-17α-methoxymethyl-17β-methoxy-estra-4,9-dien-3-on gemäß Anspruch 1 zur Anwendung als Arzneimittel.

7. Verwendung von amorphem 11β-[4E-(Hydroxyiminomethyl)phenyl]-17α-methoxymethyl-17β-methoxy-estra-4,9-dien-3-on nach Anspruch 1 zur Herstellung eines Arzneimittels für die weibliche Fertilitätskontrolle, zur Behandlung von hormonabhängigen gynäkologischen Störungen und für die Hormon-Replacement-Therapie.

## Claims

1. Amorphous 11β-[4E-(hydroxyiminomethyl)phenyl]-17α-methoxymethyl-17β-methoxy-estra-4,9-dien-3-one, **characterized in that** in the x-ray-powder diffractogram (XRPD) no crystalline reflexes are seen.

2. Crystalline solvates of 11β-[4E-(hydroxyiminomethyl)phenyl]-17α-methoxymethyl-17β-methoxy-estra-4,9-dien-3-one, namely the methyl-tert-butyl ether solvate, whereby the crystalline form is **characterized by** the x-ray-powder diffractogram that is depicted in Figure 4c;
the acetone solvate, whereby the crystalline form is **characterized by** the x-ray-powder diffractogram that is depicted in Figure 4b; and
the ethanol solvate, whereby the crystalline form is **characterized by** the x-ray-powder diffractogram that is depicted in Figure 4a.

3. Process for the production of a crystalline solvate of 11β-[4E-(hydroxyiminomethyl)phenyl]-17α-methoxymethyl-17β-methoxy-estra-4,9-dien-3-one according to claim 2 that comprises the following steps:
a) Production of a solvate of 11β-[4E-(hydroxyiminomethyl)phenyl]-17α-methoxymethyl-17β-methoxy-estra-4,9-dien-3-one and dissolution of the solvate above the saturation solubility in a solvent that is different from the solvate solvent and in which the solvate-crystal structure is unstable,
b) the optional addition of another solvent as a recrystallization inhibitor, and
c) the addition of a solvating agent-solvent and crystallization of the solvate of 11β-[4E-(hydroxyimino-methyl)phenyl]-17α-methoxymethyl-17β-methoxy-estra-4,9-dien-3-one.

4. Process for the production of amorphous 11β-[4E-(hydroxyiminomethyl)phenyl]-17α-methoxymethyl-17β-methoxy-estra-4,9-dien-3-one by drying the crystalline solvate obtained according to claim 3 below the glass transition point of the amorphous substance, whereby the drying of the solvate is carried out during the desolvation phase with a heating rate in the product of at least 0.5°C/minute or from a solution of the solvate by spray-drying.

5. Pharmaceutical composition that comprises amorphous 11β-[4E-(hydroxyiminomethyl)phenyl]-17α-methoxymethyl-17β-methoxy-estra-4,9-dien-3-one according to claim 1, in combination with a pharmaceutically compatible vehicle and/or diluent.

6. Amorphous 11β-[4E-(hydroxyiminomethyl)phenyl]-17α-methoxymethyl-17β-methoxy-estra-4,9-dien-3-one according to claim 1 for use as a pharmaceutical agent.

7. Use of amorphous 11β-[4E-(hydroxyiminomethyl)phenyl]-17α-methoxymethyl-17β-methoxy-estra-4,9-dien-3-one according to claim 1 for the production of a pharmaceutical agent for female birth control, for treating hormone-dependent gynecological disorders and for hormone replacement therapy.

## Revendications

1. 11β-[4E-(hydroxyiminométhyl)phényl]-17α-méthoxyméthyl-17β-méthoxy-estra-4,9-dién-3-one amorphe, **caractérisé en ce qu'**on ne voit aucune raie de réflexion cristalline dans le diffractogramme de poudre par rayons X (XPRD).

2. Solvates cristallins de 11β-[4E-(hydroxyiminométhyl)phényl]-17α-méthoxyméthyl-17β-méthoxy-estra-4,9-dién-3-one, à savoir le solvate avec le méthyl-tert.-butyléther, la forme cristalline étant **caractérisée par** le diffractogramme de poudre par rayons X représenté dans la figure 3c, le solvate avec l'acétone, la forme cristalline étant **caractérisée par** le diffractogramme de poudre par rayons X représenté dans la figure 3b, et le solvate avec l'éthanol, la forme cristalline étant **caractérisée par** le diffractogramme de poudre par rayons X représenté dans la figure 3a.

3. Procédé de préparation d'un solvate cristallin de 11β-[4E-(hydroxyiminométhyl)phényl]-17α-méthoxyméthyl-17β-méthoxy-estra-4,9-dién-3-one selon la revendication 2, comprenant les étapes suivantes :
a) préparation d'un solvate de 11β-[4E-(hydroxyiminométhyl)phényl]-17α-méthoxyméthyl-17β-méthoxy-estra-4,9-dién-3-one et dissolution du solvate au delà de la solubilité de saturation dans un solvant qui est différent du solvant du solvate et dans lequel la structure cristalline du solvate est instable,
b) ajout éventuel d'un autre solvant à titre d'inhibiteur de recristallisation et
c) ajout d'un solvant formant un solvate et cristallisation du solvate de 11β-[4E-(hydroxyiminométhyl)phényl]-17α-méthoxyméthyl-17β-méthoxy-estra-4,9-dién-3-one.

4. Procédé de préparation de 11β-[4E-(hydroxyiminométhyl)phényl]-17α-méthoxyméthyl-17β-méthoxy-estra-4,9-dién-3-one amorphe par séchage du solvate cristallin obtenu selon la revendication 3 au-dessus du point de transition vitreuse de la substance amorphe, moyennant quoi le séchage du solvate s'effectue durant la phase de désolvatation avec une vitesse de chauffage d'au moins 0,5 °C/min du produit ou par séchage par atomisation d'une solution du solvate.

5. Composition pharmaceutique comprenant du 11β-[4E-(hydroxyiminométhyl)phényl]-17α-méthoxyméthyl-17α-méthoxy-estra-4,9-dién-3-one amorphe selon la revendication 1 associé à un vecteur et/ou un agent diluant pharmaceutiquement compatible.

6. 11β-[4E-(Hydroxyiminométhyl)phényl]-17α-méthoxyméthyl-17β-méthoxy-estra-4,9-dién-3-one amorphe selon la revendication 1 destiné à une utilisation comme médicament.

7. Utilisation de 11β-[4E-(hydroxyiminométhyl)phényl]-17α-méthoxyméthyl-17β-méthoxy-estra-4,9-dién-3-one amorphe selon la revendication 1 pour préparer un médicament destiné au contrôle de la fertilité féminine, au traitement des troubles gynécologiques hormonodépendants et au traitement par des hormones de substitution.
